# EUROPÄISCHE PATENTSCHRIFT

(11) **EP 2 041 731 B1**
(45) Veröffentlichungstag und Bekanntmachung des Hinweises auf die Patenterteilung: **30.11.2011**
(21) Anmeldenummer: 07765028.1
(22) Anmeldetag: 03.07.2007
(51) Int. Cl.: G07D 7/14

(54) **VERFAHREN UND VORRICHTUNG ZUR AUTHENTIFIZIERUNG VON MIT EINER MARKIERUNG VERSEHENEN GEGENSTÄNDEN**
METHOD AND DEVICE FOR AUTHENTICATING OBJECTS PROVIDED WITH A MARKING
PROCÉDÉ ET DISPOSITIF D'IDENTIFICATION D'OBJETS PRÉSENTANT UN MARQUAGE

(30) Priorität: 03.07.2006 DE 102006031014
(43) Veröffentlichungstag der Anmeldung: 01.04.2009
(73) Patentinhaber: Secutech International Pte. Ltd., Singapore 069443 (SG)
(72) Erfinder: BERTLING, Wolf, M., 91056 Erlangen (DE)
(74) Vertreter: UEXKÜLL & STOLBERG
(86) Internationale Anmeldenummer: PCT/EP2007/005885
(87) Internationale Veröffentlichungsnummer: WO 2008/003465

(56) Entgegenhaltungen:
- WO-A-01/51652
- WO-A-03/038000
- DE-A- 10 105 339

## Beschreibung

Die Erfindung betrifft ein Verfahren und eine Vorrichtung zur Authentifizierung von mit einer Markierung versehenen Gegenständen.

Die WO 01/51652 A2 offenbart ein Verfahren, bei dem ein Gegenstand zur Authentifizierung mit einer fälschungssicheren Markierung versehen wird. Die Markierung besteht aus einer ersten Nukleinsäure, welche in Form erster Flächenelemente angeordnet ist. Zur Identifizierung der Markierung wird ein durch die ersten Flächenelemente gebildetes Teilmuster sichtbar gemacht. Dazu wird die erste Nukleinsäure mit einer dazu komplementären zweiten Nukleinsäure in Kontakt gebracht. Bei einer Hybridisierung der ersten und der zweiten Nukleinsäure ist eine Fluoreszenz beobachtbar. Zur Messung eines Hintergrundsignals können neben den ersten Flächenelementen zusätzlich zweite Flächenelemente vorgesehen sein, welche eine dritte Nukleinsäure enthalten. Die dritte Nukleinsäure ist nicht komplementär zur zweiten Nukleinsäure. Zwischen der zweiten und dritten Nukleinsäure tritt keine Hybridisierung auf. Eine Messung der Fluoreszenz der zweiten Flächenelemente ermöglicht eine Bestimmung der Hintergrundfluoreszenz.

Die WO 02/072878 A1 beschreibt ein Verfahren, bei dem ein Gegenstand zur Authentifizierung mit einer Markierung versehen ist. Die Markierung umfasst ein erstes und ein zweites Flächenelement. Das erste Flächenelement ist mit einer vorgegebenen ersten und das zweite Flächenelement mit einer vorgegebenen dritten Nukleinsäure imprägniert. Zur Authentifizierung des Gegenstands werden das erste und das zweite Flächenelement mit einer Nachweislösung in Kontakt gebracht. Die Nachweislösung enthält eine zur ersten Nukleinsäure komplementäre zweite Nukleinsäure, welche mit einem Fluorophor markiert ist. Infolge einer Hybridisierung der ersten und der dazu komplementären zweiten Nukleinsäure ist ein erhöhtes Fluoreszenzsignal beobachtbar. Dagegen führt ein Kontakt der zweiten und der dritten Nukleinsäure im zweiten Flächenelement zu keiner Hybridisierung. Es ist dort keine erhöhte Fluoreszenz beobachtbar. Die Messung der Fluoreszenz im ersten und zweiten Flächenelement ermöglicht eine Identifizierung der Markierung.

In der Praxis wird als zweite Nukleinsäure meist eine Nukleinsäure mit einer Haarnadelstruktur verwendet, an deren erstem freien Ende der Fluorophor und an deren gegenüberliegendem zweiten freien Ende in einem ein Fluoreszenzsignal löschenden Abstand ein Quencher gebunden sind. Beim Inkontaktbringen der zweiten Nukleinsäure mit der ersten Nukleinsäure öffnet sich wegen der angestrebten Hybridisierung die Haarnadelstruktur und die räumliche Beziehung zwischen dem Fluorophor und dem Quencher wird aufgelöst. Infolgedessen ist das Fluoreszenzsignal beobachtbar.

Die nach dem Stand der Technik bekannten Verfahren sind in mehrfacher Hinsicht nachteilig. Zur Durchführung einer exakten Messung sind stets mit unterschiedlichen Nukleinsäuren beladene Flächenelemente in der Markierung erforderlich. Die Herstellung der Markierung ist damit aufwändig. Abgesehen davon ist es zum Nachweis der Echtheit der Markierung erforderlich, sowohl die Fluoreszenz des ersten als auch des zweiten Flächenelements zu messen und auszuwerten. Schließlich kann es durch physikalische oder chemische Prozesse auch im ersten Flächenelement zu einer unspezifischen Fluoreszenz kommen, welche zu, falsch positiven oder negativen Resultaten führen kann. Beispielsweise kann das Problem auftreten, dass die angesprochene Haarnadelstruktur nicht nur durch eine Hybridisierung mit der komplementären ersten Nukleinsäure, sondern auch durch andere Einflüsse aufgehoben werden kann. Die Haarnadelstruktur kann durch eine Behandlung der zweiten Nukleinsäure mit Säuren oder Basen oder bei

Überschreiten einer bestimmten Temperatur zerstört werden. Es kann also beispielsweise durch eine Imprägnierung der Markierung mit einer Säure ein falsch positives Fluoreszenzsignal erzeugt und damit die Authentizität des Gegenstands vorgetäuscht werden.

Aufgabe der Erfindung ist es, die Nachteile nach dem Stand der Technik zu beseitigen. Es soll insbesondere ein möglichst einfach durchführbares Verfahren zur Authentifizierung von mit einer Markierung versehenen Gegenständen angegeben werden, welches sich durch eine verbesserte Fälschungssicherheit und Zuverlässigkeit auszeichnet. Ferner soll eine zur Durchführung des Verfahrens geeignete Vorrichtung angegeben werden.

Diese Aufgabe wird durch die Merkmale der Ansprüche 1 und 18 gelöst. Zweckmäßige Ausgestaltungen der Erfindung ergeben sich aus den Merkmalen der Ansprüche 2 bis 17.

Nach Maßgabe der Erfindung ist ein Verfahren zur Authentifizierung von mit einer Markierung versehenen Gegenständen, wobei die Markierung eine Markierungsnukleinsäure enthält, mit folgenden Schritten vorgesehen:
a) Bereitstellen einer Nachweislösung, welche
   a1) eine zumindest abschnittsweise doppelsträngige Nachweisnukleinsäure enthält, bei der einer der beiden Nachweisnukleinsäurestränge zur Markierungsnukleinsäure zumindest abschnittsweise komplementär ist, und wobei am einen Nachweisnukleinsäurestrang ein erster Fluorophor und am anderen Nachweisnukleinsäurestrang ein erster Quencher in einem das erste Fluoreszenzsignal des ersten Fluorophors löschenden Abstand gebunden sind/ und weiche des Weiteren
   a2) eine zumindest abschnittsweise doppelsträngige Referenznukleinsäure enthält, deren Referenznukleinsäurestränge weder zur Markierungsnukleinsäure noch zu einem der beiden Nachweisnukleinsäurestränge komplementär sind, und wobei am einen Referenznukleinsäurestrang ein vom ersten Fluorophor verschiedener zweiter Fluorophor and am anderen Referenznukleinsäurestrang ein zweiter Quencher in einem ein zweites Fluoreszenzsignal des zweiten Fluorophors löschenden Abstand gebunden sind,
b) Inkontaktbringen der Nachweislösung mit der Markierung unter für eine Hybridisierung einer der beiden Nachweisnukleinsäurestränge mit der Markierungsnukleinsäure geeigneten Bedingungen,
c) Beobachten einer von der Markierung emittierten Fluoreszenz und
d) Feststellen der Authentizität des Gegenstands, wenn (i) die beobachtete Fluoreszenz zumindest einer erwarteten ersten Eigenschaft des ersten Fluoreszenzsignals entspricht und (ii) zumindest eine erwartete zweite Eigenschaft des zweiten Fluoreszenzsignals nicht beobachtbar ist.

Der eine Nachweisnukleinsäurestrang ist zumindest soweit abschnittsweise komplementär mit der Markierungsnukleinsäure, dass bei einem Inkontaktbringen der Markierungsnukleinsäure mit dem einen Nachweisnukleinsäurestrang eine Hybridisierung unter vorgegebenen Bedingungen stattfindet. Durch die Hybridisierung des Nachweisnukleinsäurestrangs mit der Markierungsnukleinsäure wird die räumliche Beziehung zwischen dem Quencher und dem ersten Fluorophor aufgehoben. Infolgedessen kommt es bei einer Einstrahlung von Licht auf den ersten Fluorophor zu einer beobachtbaren Fluoreszenz.

Die beiden Referenznukleinsäurestränge der Referenznukleinsäure sind zur Markierungsnukleinsäure nicht komplementär. Bei Inkontaktbringen der Referenznukleinsäure mit der Markierungsnukleinsäure bleibt die doppelsträngige Struktur der Referenznukleinsäure erhalten. Ein zweites Fluoreszenzsignal ist nicht beobachtbar oder befindet sich unterhalb eines definierten Schwellwerts. Bei einer Störung der Markierung, wenn z.B. Stoffe, wie Säuren, Basen oder dgl., verunreinigt oder zum Zwecke der Fälschung aufgebracht worden sind oder das Inkontaktbringen der Nachweislösung mit der Markierung unter Bedingungen, beispielsweise einer zu hohen Temperatur, erfolgt, unter denen eine doppelsträngige Struktur zweier Nukleinsäuren aufgelöst wird, ist die erwartete zweite Eigenschaft des zweiten Fluoreszenzsignals beobachtbar. Das zeigt an, dass eine falsch positive Messung vorliegt. Es kann damit auf einfache, sichere und zuverlässige Weise eine hohe Zuverlässigkeit und Fälschungssicherheit des Verfahrens gewährleistet werden. Ein weiterer Vorteil des vorgeschlagenen Verfahrens besteht darin, dass die Identifizierung der Markierung mittels eines einzigen Flächenelements erfolgen kann.

Die Markierung ist zweckmäßigerweise fest an der Oberfläche des Gegenstands angebracht. Zur Authentifizierung des Gegenstands ist es nicht erforderlich, die Markierung zu entfernen. Vorteilhafterweise kann die Markierung an Ort und Stelle, d.h. in ihrem auf der Oberfläche des Gegenstands befestigten Zustand identifiziert werden. Abgesehen davon ist es nicht notwendig, Flüssigkeit, beispielsweise überschüssige Nachweisnukleinsäure, nach den Auftragen von der Markierung zu entfernen. Das macht das vorgeschlagene Verfahren besonders einfach durchführbar.

Nach einer vorteilhaften Ausgestaltung der Erfindung sind der erste Fluorophor und der erste Quencher im Bereich des einen Endes der Nachweisnukleinsäure gebunden. In ähnlicher Weise können der zweite Fluorophor und der zweite Quencher im Bereich des einen Endes der Referenznukleinsäure gebunden sein. Das Vorsehen der Fluorophor/Quencherpaare im Bereich des Endes der Nachweis- und/oder Referenznukleinsäure ermöglicht eine besonders einfache Herstellung derselben. Die doppelsträngige Struktur wird im Falle des vorgeschlagenen endständigen Vorsehens der Fluorophor/Quencherpaare wenig gestört.

Nach einer weiteren vorteilhaften Ausgestaltung werden die Schritte lit. b) und lit. c) zeitlich aufeinanderfolgend innerhalb von 60 Sekunden durchgeführt. Zweckmäßigerweise werden die Schritte lit. b) und lit. c) bei Umgebungstemperatur durchgeführt. Das erlaubt eine schnelle und einfache Verfahrensführung vor Ort. Die Markierung kann unmittelbar auf dem Gegenstand nachgewiesen werden. Dazu ist es insbesondere nicht erforderlich, nach dem Aufbringen der Nachweisflüssigkeit eine weitere Flüssigkeit auf die Markierung aufzubringen. Insbesondere ist es nicht erforderlich, eine Waschlösung auf die Markierung aufzutragen.

Nach einer zweckmäßigen Ausgestaltung liegt die Nachweisnukleinsäure in einer Haarnadelstruktur vor, bei welcher die Nachweisnukleinsäure zwei zueinander komplementäre Äste aufweist. In ähnlicher Weise kann auch die Referenznukleinsäure in einer Haarnadelstruktur vorliegen, bei welcher die Referenznukleinsäurestränge zwei zueinander komplementäre Äste aufweist. Die vorgeschlagenen selbst-komplementären Nachweis- und/oder Referenznukleinsäuren eignen sich in besonderer Weise zum Nachweis der Markierungsnukleinsäure oder ggf. falsch positiver Signale erzeugender Stoffe oder Bedingungen.

Nach einer weiteren Ausgestaltung wird beim Schritt lit. c) die Markierung mit Licht eines vorgegebenen Wellenlängenbereichs bestrahlt. Dabei umfasst der Wellenlängenbereich solche Wellenlängen, mit denen der erste und zweite Fluorophor zur Erzeugung eines ersten und eines zweiten Fluoreszenzsignals anregbar ist. Damit können die Intensitäten der Fluoreszenzsignale klar gegenüber dem Hintergrund erhöht und somit eine besonders sichere und zuverlässige Messung gewährleistet werden. - Zur weiteren Verbesserung der Lichtausbeute kann die Markierung auf einer Licht reflektierenden Unterlage aufgebracht sein. Zweckmäßigerweise wird die Nachweislösung auf ein einziges, die Markierungsnukleinsäure enthaltendes Nachweisfeld der Markierung aufgebracht. Das erleichtert erheblich die Beobachtung der von der Markierung emittierten Fluoreszenz mittels eines Handscanners. Es ist insbesondere nicht erforderlich, den Handscanner über ein Nachweismarkierungsfeld und ein davon räumlich getrenntes Referenzmarkierungsfeld zu führen.

Nach einer weiteren Ausgestaltung wird die Nachweislösung mit einem Stift auf die Markierung aufgetragen. Das macht die Authentifizierung des Gegenstands besonders einfach. Es muss lediglich die Markierungsfläche mit dem Stift überstrichen und nachfolgend die von der Markierung emittierte Fluoreszenz mittels einer geeigneten Vorrichtung gemessen werden.

Gemessen wird jeweils zumindest eine vorgegebene erwartete Eigenschaft der ersten und zweiten Fluoreszenzsignale. Dabei kann die erwartete erste Eigenschaft des ersten Fluoreszenzsignals eine vorgegebene erste Mindestintensität in einem ersten Wellenlängenbereich und die erwartete zweite Eigenschaft eine vorgegebene zweite Mindestintensität in einem zweiten Wellenbereich sein. Zur Feststellung der Authentizität des Gegenstands können der erste und der zweite Fluorophor sich also hinsichtlich der Wellenlänge der damit erzeugten ersten und zweiten Fluoreszenzsignale unterscheiden. Zum Feststellen der Authentizität des Gegenstands wird in den Wellenlängenbereichen des ersten und des zweiten Fluoreszenzsignals gemessen. Sofern im zweiten Wellenlängenbereich ein zweites Fluoreszenzsignal mit einer vorgegebenen Mindestintensität beobachtet wird, oder ein Verhältnis zwischen dem ersten und zweiten Fluoreszenzsignal bestimmt wird, welches außerhalb eines Erwartungswerts liegt, wird die Markierung als nicht authentifiziert bewertet.

Nach einer weiteren besonders vorteilhaften Ausgestaltung kann es sich bei der erwarteten ersten Eigenschaft auch um ein vorgegebenes Abklingverhalten des ersten Fluoreszenzsignals und die erwartete zweite Eigenschaft um ein vorgegebenen Abklingverhalten des zweiten Fluoreszenzsignals handeln. Beispielsweise kann das vom ersten Fluorophor erzeugte Fluoreszenzsignal besonders schnell abklingen, wohingegen das vom zweiten Fluorophor erzeugte zweite Fluoreszenzsignal besonders langsam abklingt. In diesem Fall kann durch eine Messung zu einem vorgegebenen Zeitpunkt nach der Anregung durch Bestrahlen der Markierung mit Licht in einem vorgegebenen Wellenlängenbereich festgestellt werden, ob das zweite Fluoreszenzsignal vorhanden ist oder nicht. Die zum vorgegebenen Zeitpunkt bzw. der vorgegebenen Abklingzeit gemessene Intensität kann auch normiert werden, indem kurz nach der Anregung zu einem früheren Zeitpunkt eine weitere Intensität gemessen und der Quotient mit der Intensität gebildet wird.

Die ersten und zweiten Fluoreszenzsignale kennen beispielsweise mit einem Fluoreszenz-Handlesegerät erfasst werden. Dazu wird das Fluoreszenz-Handlesegerät auf die Markierung aufgesetzt, Licht zur Anregung des ersten und zweiten Fluorophors erzeugt und die von der Markierung emittierte Fluoreszenz bestimmt. Es können dazu in einem jeweils vorgegebenen Wellenlängenbereich die Intensitäten der ersten und zweiten Fluoreszenzsignale erfasst werden. Zur Bestimmung der Authentizität der Markierung können die gemessenen Intensitäten mit Erwartungswerten verglichen und bei einer Übereinstimmung mit den Erwartungswerten als authentifiziert und bei einer fehlenden Übereinstimmung mit den Erwartungswerten als nicht authentifiziert bewertet werden. Vorteilhafterweise kann es sich bei dem Erwartungswert um ein Verhältnis zwischen einer erwarteten ersten und zweiten Intensität handeln. Anstelle eines Verhältnisses kann aber auch eine Differenz oder eine andere Verknüpfung erwarteter Werte bzw. Eigenschaften mit den gemessenen Eigenschaften zum Nachweis der Intensität der Markierung verwendet werden. Die vorgenannten Erwartungswerte können anhand von Serienmessungen an intakten Markierungen und an verunreinigten Markierungen gewonnen werden.

Bei dem erfindungsgemäßen Verfahren ist es vorteilhafterweise lediglich erforderlich, die von der einzigen Markierungsfläche emittierte Fluoreszenz zu beobachten. Es ist insbesondere nicht erforderlich, das Handlesegerät zur Erfassung beispielsweise einer von einer Referenzfläche emittierten Fluoreszenz über die Markierung zu bewegen. Das vereinfacht das Verfahren. - Selbstverständlich ist es auch möglich, die Markierung auf einem ersten Markierungsfeld vorzusehen und ein zweites Referenzfeld vorzusehen, welches die Markierungsnukleinsäure nicht enthält. In diesem Fall kann der Nachweis der Markierung dadurch erfolgen, dass die Nachweislösung auf das Markierungsfeld sowie auf das Referenzfeld aufgebracht wird und sowohl die vom Markierungsfeld als auch vom Referenzfeld emittierte Fluoreszenz beobachtet wird. Die vom Referenzfeld emittierte Fluoreszenz gibt die Hintergrundfluoreszenz wieder. Indem das die Hintergrundfluoreszenz wiedergebende Signal mit dem von dem Markierungsfeld emittierten Fluoreszenzsignal ins Verhältnis gesetzt wird, kann eine besonders zuverlässige Authentifizierung der Markierung erfolgen.

Vorteilhafterweise enthält die Markierung zumindest eine weitere Nukleinsäure, welche weder zu den Nachweisnukleinsäuresträngen noch zu den Referenznukleinsäuresträngen komplementär ist. Die weitere Nukleinsäure kann relativ zur Markierungsnukleinsäure im Überschuss in der Markierung enthalten sein. Die weitere Nukleinsäure dient dazu, die Markierungsnukleinsäure zu verstecken bzw. deren Isolierung aus der Markierung durch nicht autorisierte Personen unmöglich zu machen. Das erhöht weiter die Fälschungssicherheit der Markierung. Bei der weiteren Nukleinsäure kann es sich z.B. um Kalbsthymus-DNA, synthetische Oligonukleotide mit Zufallssequenzen, tRNA, Heringsperm-DNA oder Pflanzen-DNA handeln.

Nach einer weiteren Ausgestaltung der Erfindung ist vorgesehen, dass als Markierung eine die Markierungsnukleinsäure und/oder die weitere Nukleinsäure enthaltende Druckfarbe verwendet wird. Es hat sich überraschenderweise gezeigt, dass ein zuverlässiger Nachweis der Authentizität des Gegenstands auch dann erreicht werden kann, wenn die Markierungsnukleinsäure in einer Druckfarbe enthalten ist. Durch Drucken der die Markierungsnukleinsäure enthaltenden Druckfarbe kann auf besonders einfache und kostengünstige Weise eine für den Laien zunächst nicht erkennbare Markierung, beispielsweise auf Dokumenten, Geldscheinen, Tickets oder dgl., hergestellt werden.

Die Markierung kann mit einem Druckverfahren auf den zu markierenden Gegenstand oder auf ein, vorzugsweise selbstklebendes, Etikett aufgebracht werden. Das Etikett ist zweckmäßigerweise so ausgeführt, dass es nicht zerstörungsfrei vom Gegenstand gelöst werden kann.

Nach einer weiteren Ausgestaltung umfasst die Markierung eine Markierungsfläche, welche lediglich wenige Quadratmillimeter, vorzugsweise 1 bis 50 mm² groß ist. Eine solche Markierung kann kostengünstig hergestellt werden.

Zur Authentifizierung der Markierung werden zweckmäßigerweise 0,01 bis 2,0 pMol Markierungsnukleinsäure verwendet. Derart geringe Mengen an Markierungsnukleinsäure sind für Fälscher schwer zu isolieren, zu analysieren und nachzuahmen. Das gilt insbesondere dann, wenn die Markierungsnukleinsäure in einem Gemisch mit zumindest einer weiteren Nukleinsäure in der Markierung vorliegt.

Zum Nachweis der Markierungsnukleinsäure ist es lediglich erforderlich, wenige Mikroliter, bevorzugt 0,2 bis 2,0 µl, der Nachweislösung auf die Markierung aufzubringen. Es ist insbesondere nicht erforderlich, die Markierung in der Nachweisflüssigkeit unterzutauchen oder die Markierung nach dem Aufbringen der Nachweisflüssigkeit zu waschen, um zum Feststellen der Authentizität eine geeignete Fluoreszenzemission zu beobachten. Damit ist es insbesondere möglich, den Gegenstand mit der darauf angebrachten Markierung zu authentifizieren. Durch das Aufbringen der geringen Mengen an Nachweisflüssigkeit wird der Gegenstand in keiner Weise beeinflusst. Die Authentizität des Gegenstands kann also schnell und einfach festgestellt werden.

Nach weiterer Maßgabe der Erfindung ist ein die Nachweislösung enthaltender Stift vorgesehen, wobei die Nachweislösung eine zumindest abschnittsweise doppelsträngige Nachweisnukleinsäure enthält, bei der einer der beiden Nachweisnukleinsäurestränge zu einer vorgegebenen Markierungsnukleinsäure zumindest abschnittsweise komplementär ist, und wobei am einen Nachweisnukleinsäurestrang ein erster Fluorophor und am anderen Nachweisnukleinsäurestrang ein erster Quencher in einem ein erstes Fluoreszenzsignal des ersten Fluorophors löschenden Abstand gebunden sind, und welche des Weiteren eine zumindest abschnittsweise doppelsträngige Referenznukleinsäure enthält, deren Referenznukleinsäurestränge weder zur Markierungsnukleinsäure noch zu einem der beiden Nachweisnukleinsäurestränge komplementär sind, und wobei a m einen Referenznukleinsäurestrang ein vom ersten Fluorophor verschiedener zweiter Fluorophor und am anderen Referenznukleinsäurestrang ein zweiter Quencher in einem ein zweites Fluoreszenzsignal des zweiten Fluorophors löschenden Abstand gebunden sind. Zur Aufnahme der Nachweislösung kann der Stift beispielsweise nach Art eines Filzstifts ausgebildet sein. Der Stift weist einen Behälter bzw. Tank zur Aufnahme der Nachweislösung auf.

Bei dem markierten Gegenstand handelt es sich bevorzugt um Güter, Waren, Verpackungen und Dokumente. Insbesondere kann es sich dabei um Markenprodukte, Zahlungsmittel, Chipkarten oder Verpackungen dafür handeln. Der zu authentifizierende Gegenstand wird vom Hersteller in den freien Warenverkehr gegeben und unterliegt damit einer potenziellen Fälschungsgefahr. Die Authentizität des Gegenstands kann bei Bedarf unter Anwendung des erfindungsgemäßen Verfahrens sicher und zuverlässig bestimmt werden. Dazu ist es lediglich erforderlich, die beispielsweise in einem Filzstift aufgenommene Nachweislösung auf die Markierung aufzutragen und anschließend mit einem Fluoreszenz-Handscanner die von der Markierung emittierte Fluoreszenz zu beobachten, zu analysieren bzw. mit vorgegebenen Erwartungswerten zu vergleichen und infolge des Ergebnisses des Vergleichs die Authentizität der Markierung festzustellen.

Nachfolgend werden Ausführungsbeispiele der Erfindung anhand der Zeichnungen näher erläutert. Es zeigen:
- Fig. 1: schematisch eine Markierungs-, Nachweis- und eine Referenznukleinsäure,
- Fig. 2: schematisch Reaktionsvarianten beim Kontakt einer Markierungsnukleinsäure mit einer Nachweis- und Referenznukleinsäure,
- Fig. 3: schematisch die Intensität der Fluoreszenz über der Zeit für eine Markierungs- und eine Referenznukleinsäure und
- Fig. 4a bis 4c: schematisch die Intensität der Fluoreszenz über der Zeit für die Nachweis- und Referenznukleinsäuren bei mehreren Reaktionsvarianten.

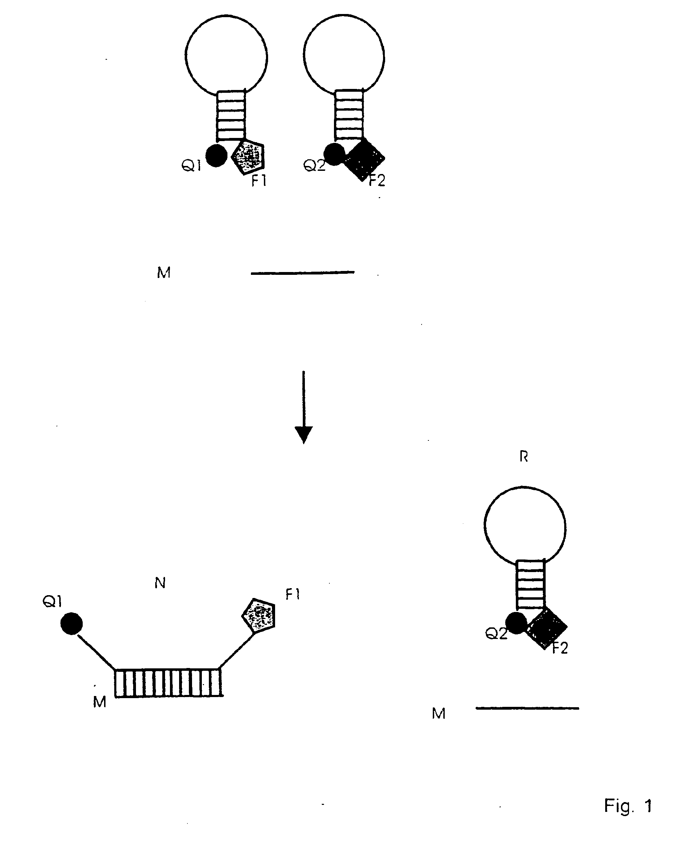

Fig. 1 zeigt schematisch eine Markierungs- M, eine Nachweis- N und eine Referenznukleinsäure R. Die Nachweisnukleinsäure N und die Referenznukleinsäure R sind jeweils nach Art eines Molecular Beacon ausgebildet. Die Nachweisnukleinsäure N umfasst eine Haarnadelstruktur, an deren freien Enden ein erstes Fluorophor F1 und ein erster Quencher Q1 in einem die Fluoreszenz des ersten Fluorophors F1 löschenden Abstand gebunden sind. Desgleichen umfasst die Referenznukleinsäure R eine Haarnadelstruktur, an deren freien Enden ein zweiter Fluorophor F2 und ein zweiter Quencher Q2 in einem die Fluoreszenz des zweiten Fluorophors F2 löschenden Abstand gebunden sind.

Die Nachweisnukleinsäure N ist zumindest abschnittsweise komplementär zur Markierungsnukleinsäure M. Beim Inkontaktbringen der Nachweisnukleinsäure N mit der Markierungsnukleinsäure M kommt es zur Hybridisierung. Der die Fluoreszenz des ersten Fluorophors F1 löschende Abstand im ersten Quencher Q1 - wird aufgehoben. Bei einer Anregung des ersten Fluorophors F1 ist eine Fluoreszenz beobachtbar. Die Referenznukleinsäure R ist dagegen nicht komplementär zur Markierungsnukleinsäure M ausgebildet. Beim Inkontaktbringen der Referenznukleinsäure R mit der Markierungsnukleinsäure M kommt es nicht zu einer Hybridisierung. Infolgedessen bleibt die räumliche Beziehung zwischen dem zweiten Quencher Q2 und dem zweiten Fluorophor F2 erhalten. Bei einer Anregung des zweiten Fluorophors F2 kommt es nicht zur Ausbildung einer Fluoreszenz.

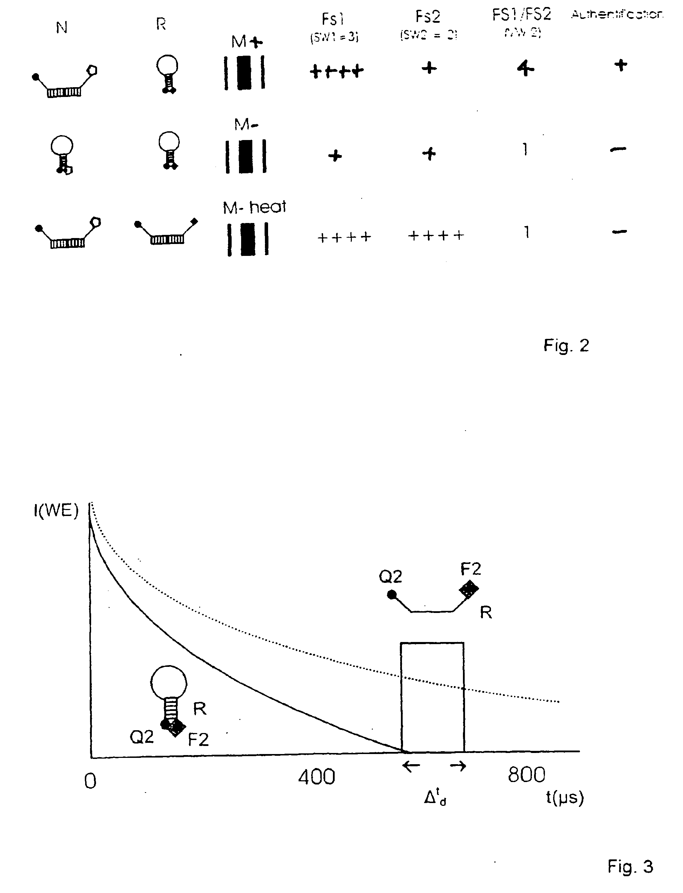

Fig. 2 zeigt in einer tabellarischen Übersicht Reaktionsvarianten beim Inkontaktbringen einer die Nachweisnukleinsäure N und die Referenznukleinsäure R enthaltenden Nachweislösung mit einer die Markierungsnukleinsäure M enthaltenen Markierung. In der ersten Zeile der Tabelle ist der Fall gezeigt, bei dem die Markierung die Markierungsnukleinsäure M enthält, gleichzeitig aber frei von eventuellen Störstoffen ist. In diesem Fall hybridisiert die Nachweisnukleinsäure N mit der Markierungsnukleinsäure M. Bei der Anregung des ersten Fluorophors F1 und des zweiten Fluorophors F2 ist insbesondere eine hohe Fluoreszenzintensität des ersten Fluorophors F1 beobachtbar. Die Fluoreszenzintensität des zweiten Fluorophors F2 ist dagegen nicht oder nur schwach beobachtbar. Die Markierung wird in diesem Fall als authentisch angesehen.

Bei der in der zweiten Zeile gezeigten Reaktionsvariante enthält die Markierung die Markierungsnukleinsäure M nicht. In diesem Fall werden weder die Haarnadelstruktur der Nachweisnukleinsäure N noch der Referenznukleinsäure R aufgelöst. Bei der Anregung der Fluorophore F1, F2 ist lediglich ein schwaches Fluoreszenzsignal beobachtbar. Die Markierung wird in diesem Fall als nicht authentisch angesehen.

Bei der in der dritten Zeile gezeigten Reaktionsvariante enthält die Markierung wiederum nicht die Markierungsnukleinsäure M. Allerdings befindet sich die Markierung hier auf einer unzulässig hohen Temperatur und/oder enthält Störstoffe. Infolgedessen werden sowohl die Haarnadelstruktur der Nachweisnukleinsäure N als auch der Referenznukleinsäure R aufgelöst. Bei einer Anregung der Fluorophore F1, F2 ist eine besonders hohe Fluoreszenzintensität beobachtbar. In diesem Fall wird die Markierung ebenfalls als nicht authentisch angesehen.

Fig. 3 zeigt die Intensität der Fluoreszenz eines Europium-Chelats über der Zeit. Ein solches Europium-Chelat kann beispielsweise als zweiter Fluorophor F2 bei der Referenznukleinsäure R verwendet werden. Als zweiter Quencher Q2 kann hier ROX verwendet werden. - Bei geschlossener Haarnadelstruktur klingt bei einer Anregung des zweiten Fluorophors F2 die emittierte Fluoreszenz, welche etwa bei 645 nm liegt, relativ schnell ab (siehe durchgezogene Kurve). Die Abklingzeit beträgt hier etwa 500 bis 600 µs. Bei geöffneter Haarnadelstruktur klingt die Fluoreszenz dagegen wesentlich langsamer ab (siehe unterbrochene Kurve). Bei einer Beobachtung der Intensität in einem Zeitintervall Δt_{d} im Bereich von 600 bis 700 µs kann so auf einfache Weise ermittelt werden, ob die Referenznukleinsäure R mit einer geschlossenen oder offenen Haarnadelstruktur vorliegt. Auf diese Weise kann die in Fig. 2 gezeigte dritte Reaktionsvariante erkannt werden.

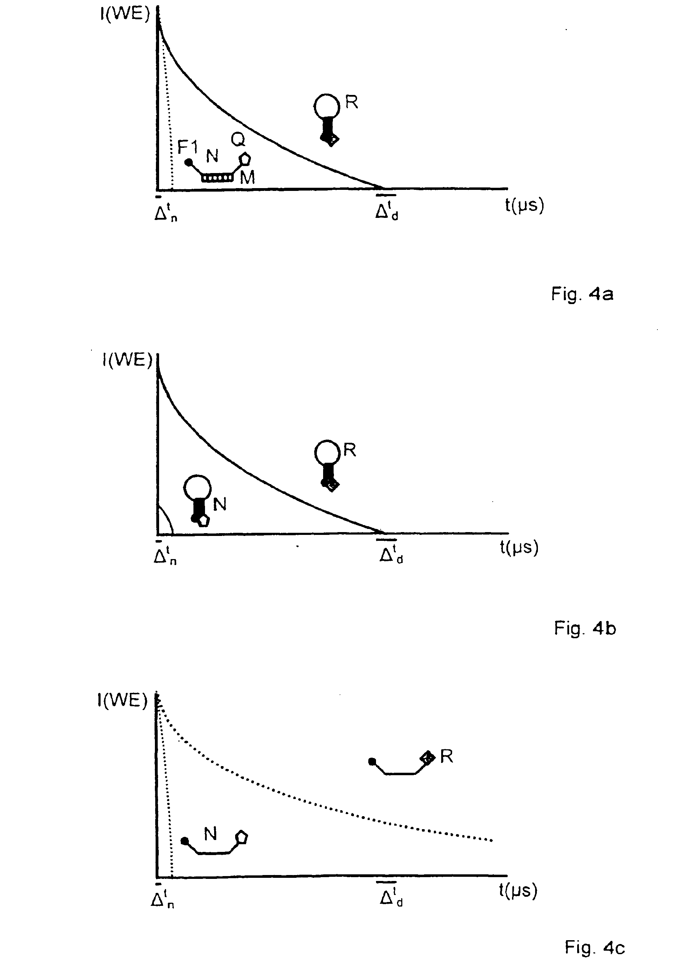

Die Fig. 4a bis 4b zeigen, wie anhand einer Beobachtung der Intensitäten in vorgegebenen Zeitintervallen festgestellt werden kann, ob die Markierung als authentisch oder nicht authentisch anzusehen ist. Beobachtet wird die Intensität der Fluoreszenz in einem ersten Zeitintervall Δtₙ bei eingeschalteter Anregungslichtquelle oder bis zu einer Abklingzeit nach Erlöschen der Anregungslichtquelle von höchstens 200 µs erstreckt. Ferner wird die Intensität der Fluoreszenz im zweiten Zeitintervall Δt_{d} gemessen, welches sich in einem Zeitfenster zwischen 600 und 800 µs nach dem Abschalten der Anregungslichtquelle befindet. Das in der Nachweisnukleinsäure N enthaltene erste Fluorophor F1 weist eine besonders kurze Abklingzeit von beispielsweise weniger als 200 µs auf. Das in der Referenznukleinsäure R enthaltene zweite Fluorophor weist eine lange Abklingzeit von mehr als 300 µs auf.

Bei dem in Fig. 4a gezeigten Beispiel enthält die Markierung die Markierungsnukleinsäure M. Störstoffe liegen nicht vor. Infolgedessen ist im ersten Zeitintervall Δtₙ eine hohe Intensität und im zweiten Zeitintervall Δt_{d} eine geringe Intensität beobachtbar. Die Markierung wird in diesem Fall als authentisch angesehen.

Bei dem in Fig. 4b gezeigten Fall liegt in der Markierung die Markierungsnukleinsäure M nicht vor. Infolgedessen wird die räumliche Struktur der Nachweisnukleinsäure N nicht geändert. Die im ersten Zeitintervall Δtₙ beobachtbare Intensität ist gering. Desgleichen ist die im zweiten Zeitintervall Δtₙ beobachtbare Intensität ebenfalls gering. In diesem Fall wird die Markierung als nicht authentisch angesehen.

Bei dem in Fig. 4c gezeigten Fall ist die Markierungsnukleinsäure M in der Markierung enthalten. Desgleichen enthält die Markierung aber Störstoffe, beispielsweise Säuren oder Basen, weiche eine Zerstörung der Haarnadelstruktur bewirken. In diesem Fall ist sowohl im ersten Zeitintervall Δtₙ als auch im zweiten Zeitintervall Δt_{d} eine hohe Intensität beobachtbar. Die Markierung wird auch in diesem Fall als nicht authentisch angesehen.

### Beispiel 1:

### Herstellung der Markierung

In einer herkömmlichen Druckerfarbe (Epson-Patrone Yellow, Refill-Kit Yellow 50 ml, Best-Nummer: EPSONY TintenCenter.com) ist als Markierungsnukleinsäure eine Nukleinsäure gemäß Sequenzprotokoll Nr. 1 (5'-AAGCCTGGAGGGATGATACTTTGCGCTTGG-3') in einer Konzentration von 1 µMol/l aufgenommen. Die Markierungsnukleinsäure wurde mittels Standard-Amidit-Festphasensynthese hergestellt.

Die Druckfarbe ist z.B. im Inkjet-Druckverfahren auf einen Träger, z.B weißes Papier, eine geeignete Kunststofffolie oder dgl., mit einem Drucker, z.B. mittels Epson Stylus D68 Photo Farbtintenstrahldrucker, auf eine Fläche von 1 mm x 2 mm aufgedruckt. Aus einem solchermaßen bedruckten Träger kann ein, vorzugsweise selbstklebendes, Etikett hergestellt werden.

Es wurden Kontrollmarkierungen ohne Markierungsnukleinsäure durch Verdrucken der Druckerfarbe ohne Zusatz von Markierungsnukleinsäure hergestellt.

### Nachweis der Markierung

Zur Authentifizierung des mit der Markierung markierten Gegenstands wurde auf zehn Markierungen eine Nachweisflüssigkeit aufgetragen. Die Nachweisflüssigkeit war zu diesem Zweck in einem Filzstift aufgenommen, der bei Kontakt mit der Markierung Nachweisflüssigkeit auf die Markierung überträgt. Bei der Nachweisflüssigkeit handelt es sich um eine wässrige Flüssigkeit, in der als Nachweisnukleinsäure eine Nukleinsäure gemäß Sequenzprotokoll Nr. 2,5-ROX-CCAAGCGCAAAGTATCATCCCTCCAGGCTTGG-DABCYL- und als Referenznukleinsäure eine Nukleinsäure gemäß Sequenzprotokoll Nr. 3 (5'-FAM-CCG AGC CAC CAA AAA TGA TAT GCT CGG-3'-DABCYL) in einer Konzentration von je 1 µMol/l in TEN (10 mM TrisCl, pH 8; 1 mM EDTA, 100 mM NaCl) enthalten ist. An den freien Enden der Nachweisnukleinsäure ist ein erstes Fluorophor/Quencherpaar in einem Abstand gebunden, welcher ein vom ersten Fluorophor erzeugtes Fluoreszenzsignal löscht. Ein erstes Fluorophor kann beispielsweise eine Rhodamingruppe oder ein Derivat davon sein; ein erster Quencher kann beispielsweise Dabcyl sein. Weitere geeignete Kombinationen für Fluorophor/Quencherpaare sind z.B. in Tyagi S, Bratu DP, and Kramer FR (1998) "Multicolor molecular beacons for allele discrimination"; Nat Biotechnol 16, 49-53 offenbart.

An der Referenznukleinsäure sind im Bereich der freien ersten Enden ein zweiter Fluorophor und ein dazu korrespondierender zweiter Quencher in einem ein zweites Fluoreszenzsignal löschenden Abstand gebunden. Bei dem zweiten Fluorophor kann es sich um eine Fluoresceingruppe oder ein Derivat davon handeln und bei dem zweiten Quencher um Dabcyl oder einen Blackhole-Quencher (Jena Bioscience GmbH, Loebstedter Strasse 80, D-07749 Jena, Germany) handeln.

Die Nachweis- und Referenznukleinsäuren wurden mittels Standard-Amidit-Festphasensynthese hergestellt. Sowohl die Nachweisnukleinsäure als auch die Referenznukleinsäure sind im vorliegenden Beispiel terminal selbst-komplementär ausgebildet und liegen in einer Haarnadelstruktur vor.

Beim Inkontaktbringen der Nachweisflüssigkeit mit der Markierungsnukleinsäure kommt es zu einer Hybridisierung zwischen der Nachweisnukleinsäure und der Markierungsnukleinsäure. Infolge dessen wird die doppelsträngige Struktur der Nachweisnukleinsäure aufgelöst und damit die ursprüngliche räumliche Beziehung zwischen dem ersten Fluorophor und dem ersten Quencher aufgehoben. Bei einer Anregung mit Licht einer Wellenlänge mit 555 nm ist ein erstes Fluoreszenzsignal bei 575 nm beobachtbar, welches durch die Fluoreszenz der Markierungsnukleinsäure hervorgerufen wird. Gleichzeitig oder unmittelbar nach der Beobachtung des ersten Fluoreszenzsignals ist bei einer Anregung mit Licht einer Wellenlänge mit 492 nm ein zweites Fluoreszenzsignal bei 517 nm beobachtbar, welches durch die Fluoreszenz der Referenznukleinsäure hervorgerufen wird. Die Messung erfolgt bevorzugt wenige Sekunden bis Minuten nach dem Kontakt mit der Nachweisflüssigkeit. Die Messung kann mit Hilfe eines Handfluoreszenz-Scanners durchgeführt werden. Das ermöglicht einen Nachweis auf dem markierten Objekt. Handfluoreszenz-Scanner mit den geforderten Eigenschaften können bei der identif GmbH, Erlangen erworben werden.

**Tabelle: 1**

| Referenznukleinsäure | | | | | | |
|---|---|---|---|---|---|---|
| Markierung | + M-NS | | - M-NS | | - M-NS + NaOH | |
| | N-NS | Ref-NS | N-NS | Ref-NS | N-NS | Ref-NS |
| 1 | 476 | 166 | 231 | 167 | 555 | 642 |
| 2 | 488 | 176 | 200 | 166 | 567 | 638 |
| 3 | 489 | 179 | 216 | 188 | 539 | 655 |
| 4 | 501 | 166 | 222 | 173 | 554 | 649 |
| 5 | 486 | 180 | 217 | 169 | 566 | 645 |
| 6 | 490 | 185 | 221 | 168 | 539 | 650 |
| 7 | 488 | 169 | 215 | 175 | 565 | 639 |
| 8 | 477 | 181 | 223 | 165 | 567 | 650 |
| 9 | 499 | 188 | 209 | 188 | 559 | 652 |
| 10 | 479 | 192 | 207 | 176 | 563 | 641 |
| Mittelwert | 487 | 178 | 216 | 173 | 557 | 646 |
| Erwartungswert | > 400 | < 400 | | | | |

| | | | | | | |
|---|---|---|---|---|---|---|
| N-NS = Fluoreszenzsignal der Nachweisnukleinsäure Ref = Fluoreszenzsignal der Referenznukleinsäure | | | | | | |

Tabelle 1 zeigt die Auswertung von zehn Markierungen nach der Auftragung der Nachweis- und Referenznukleinsäuren. In den Spalten N-NS ist der Wert Fluoreszenz der Nachweisnukleinsäure bei 575 nm angegeben. In den Spalten Ref-NS ist der Wert Fluoreszenz der Referenznukleinsäure bei 517 nm angegeben. In den Spalten + M-DNA wurden gedruckte Markierungen mit Markierungsnukleinsäure verwendet.

Der Wert für die Nachweisnukleinsäure (N-NS) liegt in einem engen Bereich um den Mittelwert von 487. Dieser Wert entspricht der geöffneten Struktur der Nachweisnukleinsäure, in der erster Fluorophor und erster Quencher durch die Hybridisierung mit der Markierungsnukleinsäure räumlich getrennt vorliegen. - Der Wert für die Referenznukleinsäure (Ref-NS) liegt in einem engen Bereich um den Mittelwert von 178. Dieser Wert entspricht der geschlossenen Struktur der Referenznukleinsäure, in der zweiter Fluorophor und zweiter Quencher durch die Rückfaltung räumlich nah benachbart vorliegen.

In den Spalten - M-DNA wurden gedruckte Markierungen ohne Markierungsnukleinsäure verwendet. Der Wert für die Nachweisnukleinsäure (N-NS) liegt in einem Bereich um den Mittelwert von 216. Dieser Wert entspricht der geschlossenen Struktur der Markierungsnukleinsäure, in der erster Fluorophor und erster Quencher bei Abwesenheit einer komplementären Markierungsnukleinsäure durch die Rückfaltung räumlich nah benachbart vorliegen. - Der Wert für die Referenznukleinsäure (Ref-NS) liegt hier wiederum in einem engen Bereich um den Mittelwert von 173. Dieser Wert entspricht der geschlossenen Struktur der Referenznukleinsäure, in welcher der zweite Fluorophor (Eu-Chelat) und zweiter Quencher (FAM) durch die Rückfaltung räumlich nah benachbart vorliegen.

In den Spalten - M-DNA + NaOH wurden gedruckte Markierungen ohne Markierungsnukleinsäure verwendet, denen vor dem Kontakt mit der Nachweisflüssigkeit 0,5 µl einer 1 molaren NaOH Lösung zugefügt wurden. Das Zufügen soll hier eine mögliche Störung der Markierung simulieren. Der Wert für die Nachweisnukleinsäure (N-NS) liegt in einem Bereich um einen Mittelwert von 557. Dieser Wert entspricht der geöffneten Struktur der Nachweisnukleinsäure, in der erster Fluorophor und erster Quencher durch die Denaturierung auf Grund des alkalischen pHs räumlich getrennt vorliegen. - Der Wert für die Referenznukleinsäure (Ref-NS) liegt in einem engen Bereich um einen Mittelwert von 664. Dieser Wert entspricht der geöffneten Struktur der Referenznukleinsäure, in welcher der zweite Fluorophor und der zweite Quencher durch die Denaturierung durch alkalischen pH räumlich getrennt vorliegen.

Die deutliche Trennung der Fluoreszenz-Werte bei An- und Abwesenheit der Markierungsnukleinsäure ermöglicht eine Festlegung von Erwartungswerten zur Beurteilung der Authentizität der Markierung. Beispielsweise kann für die Bewertung der Markierung ein Erwartungswert von über 400 für die Nachweisnukleinsäure und ein Erwartungswert von unter 400 für die Referenznukleinsäure festgelegt werden. Beide Kriterien müssen für eine Bewertung der Markierung als authentisch erfüllt werden. Nach diesen Kriterien sind alle Markierungen, welche Markierungsnukleinsäure enthalten (Tabelle 1, Spalten + M-NS) als authentisch bewertet, da sämtliche Werte für die Nachweisnukleinsäure oberhalb von 400 liegen und sämtliche Werte für die Referenznukleinsäure unterhalb von 400 liegen. Ein Fehlen der Markierungsnukleinsäure (Tabelle 1, Spalten -M-NS) führt zu Werten unterhalb von 400 für die Nachweis- und Referenznukleinsäure, damit liegt der Wert die Nachweisnukleinsäure unterhalb des Erwartungswertes. Die Kriterien für die Authentizität der Markierung sind nicht erfüllt.

Bei Anwesenheit von Störstoffen in der Markierung erhöht sich der Wert der Fluoreszenz der Referenznukleinsäure auf Werte oberhalb des Erwartungswerts für die Referenznukleinsäure (Tabelle 1, Spalte - M-NS, +NaOH). Das Überschreiten des Erwartungswerts für die Referenznukleinsäure führt ebenfalls zu einer Bewertung der Markierung als nicht authentisch.

Zur weiteren Vereinfachung des Verfahrens ist es möglich, ein erstes und zweites Fluorophor zu verwenden, die mit der gleichen Wellenlänge angeregt werden können und deren Emission bei unterschiedlichen Wellenlängen getrennt detektiert werden können. Geeignete Fluorophore sind beispielsweise bei Tyagi S, Marras SAE, and Kraxer FR (2CC0; "Wavelengtn-shifting molecular beacons"; Nat Biotechnol 19, 1191-1196 beschrieben.

### Beispiel 2:

Es wurden die gleichen Oligonukleotide wie im Beispiel 1 verwendet, nämlich als Markierungsnukleinsäure (Sequenzprotokoll Nr. 1 (5'-AAGCCTGGAGGGATGATACTTTGCGCTTGG-3') als und Nachweisnukleinsäure (Sequenzprotokoll Nr. 2,5-ROX-CCAAGCGCAAAGTATCATCCCTCCAGGCTTGG-DABCYL-3'). Als Referenznukleinsäure wurde eine Oligonukleotid mit einer Rückfaltung verwendet, dessen 5'-Ende mit einer Europium-Chelat-Gruppe und dessen 3'-Ende mit einer FAM-Gruppe markiert war (Sequenzprotokoll Nr. 4,5'-Europium-Chelat-CCG AGC CAC CAA AAA TGA TAT GCT CGG-FAM-3'). Die FAM-Gruppe war mittels Standard-Amidit-Chemie während der Oligonukleotid-Synthese eingeführt. Die Europium-Chelat-Gruppe wurde an eine terminale Aminogruppe nach der Oligonukleotid-Synthese gekoppelt. Die Eu-Kopplung erfolgte mit dem Eu-Labeling-Kit von Perkin-Elmer (Produktnummer C401-101) nach Angaben des Herstellers. Die Referenz- und Nachweisnukleinsäuren wurden in einer Konzentration von je 1 µMol/l in TEN-Puffer aufgenommen und in einen Filzstift gefüllt.

Gedruckte Markierungen wurden wie beim Beispiel 1 mit der Referenz- und Nachweisnukleinsäure in Kontakt gebracht. Das Fluoreszenzsignal der Nachweisnukleinsäure wurde wie im Beispiel 1 mit einem Handfluoreszenz-Scanner bestimmt. Das Fluoreszenzsignal der Referenznukleinsäure wurde mittels eines Fluoreszenz-Scanners mit zeitverzögerter Detektion (Victor - Multilabel counter, Perkin Eimer, Produktnummer 1420) bestimmt. Die Fluoreszenz der Referenznukleinsäure wurde bei einer Anregungswellenlänge von 340 nm und einer Emissionswellenlänge von 613 nm mit einer Verzögerungszeit von 500 µs gemessen.

**Tabelle: 2**

| Referenznukleinsäure mit langsamen Fluoreszenz Abklingverhalten | | | | | | |
|---|---|---|---|---|---|---|
| Markierung | + M-DNA | + M-DNA | - M-DNA | - M-DNA | - M-DNA + NaOH | - M-DNA + NaOH |
| | N-NS | Ref-NS | N-NS | Ref-NS | N-NS | Ref-NS |
| 1 | 477 | 2913 | 211 | 2616 | 545 | 10443 |
| 2 | 484 | 2793 | 230 | 2699 | 577 | 10875 |
| 3 | 485 | 2791 | 222 | 2779 | 553 | 11755 |
| 4 | 475 | 2699 | 210 | 2869 | 552 | 11666 |
| 5 | 476 | 2892 | 231 | 2689 | 549 | 10752 |
| 6 | 491 | 2870 | 236 | 2787 | 549 | 10998 |
| 7 | 479 | 2684 | 216 | 2778 | 561 | 10632 |
| 8 | 488 | 2932 | 221 | 2793 | 571 | 11321 |
| 9 | 489 | 2879 | 211 | 2887 | 553 | 10962 |
| 10 | 478 | 2771 | 209 | 2777 | 553 | 11133 |
| Mittelwert | 482 | 2822 | 219 | 2767 | 556 | 11053 |
| Schwellenwert | > 400 | < 5000 | | | | |

Tabelle 2 zeigt die Auswertung von zehn Markierungen nach dem Auftragen der Nachweis- und Referenznukleinsäuren. In den Spalten N-NS ist der Wert Fluoreszenz der Nachweisnukleinsäure bei 575 nm angegeben. In den Spalten Ref-NS ist der Wert Fluoreszenz der Referenznukleinsäure bei 613 nm angegeben.

In den Spalten + M-DNA. wurden gedruckte Markierungen mit Markierungsnukleinsäure verwendet. Der Wert für die Nachweisnukleinsaure (N-NS) liegt in einem engen Bereich um einen Mittelwert von 482. Dieser Wert entspricht der geöffneten Struktur der Nachweisnukleinsäure, in welcher der erste Fluorophor und der erste Quencher durch die Hybridisierung mit der Markierungsnukleinsäure räumlich getrennt vorliegen.

Der Wert für die Referenznukleinsäure (Ref-N5) liegt in einem engen Bereich, um den Mittelwert von 2822. Dieser Wert entspricht der geschlossenen Struktur der Referenznukleinsäure, in welcher der zweite Fluorophor (Eu-Chelat) und der zweite Quencher (FAM) durch die Rückfaltung räumlich nah benachbart vorliegen. Durch die räumliche Nachbarschaft kommt es zu einer Unterdrückung der Fluoreszenz des Eu-Chelats und/oder zu einer Verkürzung der Lebensdauer der Fluoreszenz bzw. des Fluoreszenzsignals. Solche Veränderungen können mit Hilfe einer zeitversetzten Erfassung der Fluoreszenz extrem empfindlich detektiert werden.

In den Spalten - M-DNA wurden gedruckte Markierungen ohne Markierungsnukleinsäure verwendet. Der Wert für die Nachweisnukleinsäure (N-NS) liegt in einem Bereich um den Mittelwert von 219. Dieser Wert entspricht der geschlossenen Struktur der Markierungsnukleinsäure, in welcher der erste Fluorophor und der erste Quencher bei Abwesenheit einer komplementären Markierungsnukleinsäure durch die Rückfaltung räumlich nah benachbart vorliegen. Der Wert für die Referenznukleinsäure (Ref-NS) liegt hier wiederum in einem engen Bereich um einen Mittelwert von 2767. Dieser Wert entspricht der geschlossenen Struktur der Referenznukleinsäure, in welcher der zweite Fluorophor (Eu-Chelat) und der zweite Quencher (FAM) durch die Rückfaltung räumlich nahe benachbart vorliegen.

In den Spalten -M-DNA + NaOH wurden gedruckte Markierungen ohne Markierungsnukleinsäure verwendet, denen vor dem Kontakt mit der Nachweisflüssigkeit 0,5 µl einer 1 molaren NaOH-Lösung zugefügt wurden. Das Zufügen soll hier eine mögliche Störung der Markierung simulieren.

Der Wert für die Nachweisnukleinsäure (N-NS) liegt in einem Bereich um einen Mittelwert von 556. Dieser Wert entspricht der geöffneten Struktur der Nachweisnukleinsäure, in welcher der erste Fluorophor und der erste Quencher durch die Denaturierung durch alkalischen pH räumlich getrennt vorliegen. - Der Wert für die Referenznukleinsäure (Ref-NS) liegt in einem engen Bereich um einen Mittelwert von 11053. Dieser Wert entspricht der geöffneten Struktur der Referenznukleinsäure, in welcher der zweite Fluorophor und der zweite Quencher durch die Denaturierung durch alkalischen pH räumlich getrennt vorliegen.

Die deutliche Trennung der Fluoreszenz-Werte bei An- und Abwesenheit der Markierungsnukleinsäure ermöglicht eine Festlegung von Erwartungswerten zur Beurteilung der Authentizität der Markierung. Beispielsweise kann für die Bewertung der Markierung ein Erwartungswert von über 400 für die Nachweisnukleinsäure und ein Erwartungswert von unter 5000 für die Referenznukleinsäure festgelegt werden. Beide Kriterien müssen für eine Bewertung der Markierung als authentisch erfüllt werden. Nach diesen Kriterien werden alle Markierungen, die Markierungsnukleinsäure enthalten (Tabelle 2, Spalten + -M-NS) als authentisch bewertet, da sämtliche Werte für die Nachweisnukleinsäure oberhalb von 400 liegen und sämtliche Werte für die Referenznukleinsäure unterhalb von 5000 liegen.

Ein Fehlen der Markierungsnukleinsäure (Tabelle 2, Spalten -M-NS) führt zu Werten unterhalb von 400 für die Nachweis- und Referenznukleinsäure. Damit liegt der Wert für die Nachweisnukleinsäure unterhalb des Erwartungswertes. Die Kriterien für die Authentizität der Markierung sind nicht erfüllt und damit die Markierungen als nicht authentisch zu bewerten.

Bei der Anwesenheit von Störstoffen in der Markierung erhöht sich der Wert der Fluoreszenz der Referenznukleinsäure auf Werte oberhalb des Erwartungswerts für die Referenznukleinsäure (Tabelle 2, Spalte - M-NS, + NaOH). Das Überschreiten des Erwartungswerts für die Referenznukleinsäure führt ebenfalls zu einer Bewertung der Markierung als nicht authentisch.

## Patentansprüche

1. Verfahren zur Authentifizierung von mit einer Markierung versehenen Gegenständen, wobei die Markierung eine Markierungsnukleinsäure enthält, mit folgenden Schritten:
a) Bereitstellen einer Nachweislösung, welche
a1) eine zumindest abschnittsweise doppelsträngige Nachweisnukleinsäure enthält, bei der einer der beiden Nachweisnukleinsäurestränge zur Markierungsnukleinsäure zumindest abschnittsweise komplementär ist, wobei am einen Nachweisnukleinsäurestrang ein erster Fluorophor und am anderen Nachweisnukleinsäurestrang ein erster Quencher in einem ein erstes Fluoreszenzsignal des ersten Fluorophors löschenden Abstand gebunden sind, und wobei durch eine Hybridisierung des Nachweisnukleinsäurestrangs mit der Markierungsnukleinsäure die räumliche Beziehung zwischen dem Quencher und dem ersten Fluorophor aufgehoben wird, und
a2) eine zumindest abschnittsweise doppelsträngige Referenznukleinsäure enthält, deren Referenznukleinsäurestränge weder zur Markierungsnukleinsäure noch zu einem der beiden Nachweisnukleinsäurestränge komplementär sind, und wobei am einen Referenznukleinsäurestrang ein vom ersten Fluorophor verschiedener zweiter Fluorophor und am anderem Referenznukleinsäurestrang ein zweiter Quencher in einem ein zweites Fluoreszenzsignal des zweiten Fluorophors löschenden Abstand gebunden sind,
b) Inkontaktbringen der Nachweislösung mit der Markierung unter für eine Hybridisierung einer der beiden Nachweisnukleinsäurestränge mit der Markierungsnukleinsäure geeigneten Bedingungen,
c) Beobachten einer von der Markierung emittierten Fluoreszenz und
d) Feststellen der Authentizität des Gegenstands, wenn (i) die beobachtete Fluoreszenz zumindest einer erwarteten ersten Eigenschaft des ersten Fluoreszenzsignals entspricht und (ii) zumindest eine erwartete zweite Eigenschaft des zweiten Fluoreszenzsignals nicht beobachtbar ist.

2. Verfahren nach Anspruch 1, wobei der erste Fluorophor und der erste Quencher im Bereich des einen Endes der Nachweisnukleinsäure gebunden sind.

3. Verfahren nach einem der vorhergehenden Ansprüche, wobei der zweite Fluorophor und der zweite Quencher im Bereich des einen Endes der Referenznukleinsäure gebunden ist.

4. Verfahren nach einem der vorhergehenden Ansprüche, wobei der Schritt lit. d) innerhalb von 60 Sekunden nach dem Schritt lit. b) durchgeführt wird.

5. Verfahren nach einem der vorhergehenden Ansprüche, wobei die Schritte lit. b) und c) bei Umgebungstemperatur durchgeführt werden.

6. Verfahren nach einem der vorhergehenden Ansprüche, wobei die Nachweisnukleinsäure in einer Haarnadelstruktur vorliegt, bei welcher die Nachweisnukleinsäure zwei zueinander komplementäre Äste aufweist.

7. Verfahren nach einem der vorhergehenden Ansprüche, wobei die Referenznukleinsäure in einer Haarnadelstruktur vorliegt, bei welcher die Referenznukleinsäurestränge zwei zueinander komplementäre Äste aufweist.

8. Verfahren nach einem der vorhergehenden Ansprüche, wobei beim Schritt lit. c) die Markierung mit Licht eines vorgegeben Wellenlängenbereichs bestrahlt wird.

9. Verfahren nach einem der vorhergehenden Ansprüche, wobei die Markierung auf einer Licht reflektierenden Unterlage aufgebracht ist.

10. Verfahren nach einem der vorhergehenden Ansprüche, wobei die Nachweislösung auf ein einziges, die Markierungsnukleinsäure enthaltendes Nachweisfeld der Markierung aufgebracht wird.

11. Verfahren nach einem der vorhergehenden Ansprüche, wobei die Nachweislösung mit einem Stift auf die Markierung aufgetragen wird.

12. Verfahren nach einem der vorhergehenden Ansprüche, wobei die erwartete erste Eigenschaft eine vorgegebene erste Mindestintensität in einem ersten Wellenlängenbereich und die erwartete zweite Eigenschaft eine vorgegebene zweite Mindestintensität in einem zweiten Wellenlängenbereich ist.

13. Verfahren nach einem der vorhergehenden Ansprüche, wobei die erwartete erste Eigenschaft ein vorgegebenes Abklingverhalten des ersten Fluoreszenzsignals und die erwartete zweite Eigenschaft ein vorgegebenes Abklingverhalten des zweiten Fluoreszenzsignals ist.

14. Verfahren nach einem der vorhergehenden Ansprüche, wobei der zweite Fluorophor ein zu einem vorgegebenen Abklingzeitpunkt eine höhere Intensität als der erste Fluorophor aufweist.

15. Verfahren nach einen der vorhergehenden Ansprüche, wobei die Markierung außer der Markierungsnukleinsäure zumindest eine weitere Nukleinsäure enthält.

16. Verfahren nach einem der vorhergehenden Ansprüche, wobei als Markierung eine die Markierungsnukleinsäure und/oder die weitere Nukleinsäure enthaltende Druckfarbe verwendet wird.

17. Verfahren nach einem der vorhergehenden Ansprüche, wobei die Markierung mit einem Druckverfahren auf den zu markierenden Gegenstand oder auf ein, vorzugsweise selbstklebendes, Etikett aufgebracht wird.

18. Stift, enthaltend eine Nachweislösung,
wobei die Nachweislösung eine zumindest abschnittsweise doppelsträngige Nachweisnukleinsäure enthält, bei der einer der beiden Nachweisnukleinsäurestränge zu einer vorgegebenen Markierungsnukleinsäure zumindest abschnittsweise komplementär ist, und wobei am einen Nachweisnukleinsäurestrang ein erster Fluorophor und am anderen Nachweisnukleinsäurestrang ein erster Quencher in einem ein erstes Fluoreszenzsignal des ersten Fluorophors löschenden Abstand gebunden sind, und wobei durch eine Hybridisierung des Nachweisnukleinsäurestrangs mit der Markierungsnukleinsäure die räumliche Beziehung zwischen dem Quencher und dem ersten Fluorophor aufgehoben wird, und wobei die Nachweislösung des Weiteren eine zumindest abschnittsweise doppelsträngige Referenznukleinsäure enthält, deren Referenznukleinsäurestränge weder zur Markierungsnukleinsäure noch zu einem der beiden Nachweisnukleinsäurestränge komplementär sind, und wobei am einen Referenznukleinsäurestrang ein vom ersten Fluorophor verschiedener zweiter Fluorophor und am anderen Referenznukleinsäurestrang ein zweiter Quencher in einem ein zweites Fluoreszenzsignal des zweiten Fluorophors löschenden Abstand gebunden sind.

## Claims

1. Method for authenticating objects provided with a mark, said mark comprising a mark nucleic acid, comprising:
a) providing a detection solution containing
a1) an at least in sections double-stranded detection nucleic acid in which one of the two detection nucleic acid strands is at least in sections complementary to the mark nucleic acid, with a first fluorophore being bound to one detection nucleic acid strand and a first quencher being bound to the other detection nucleic acid strand at a distance which causes a first fluorescence signal of the first fluorophore to be quenched, and wherein said hybridization of the detection nucleic acid strand with the mark nucleic acid abolishes the spatial relationship between the quencher and the first fluorophore;
a2) an at least in sections double-stranded reference nucleic acid whose reference nucleic acid strands are complementary to neither the mark nucleic acid nor either of the two detection nucleic acid strands, with a second fluorophore which is different from the first fluorophore being bound to one reference nucleic acid strand and a second quencher being bound to the other reference nucleic acid strand at a distance which causes a second fluorescence signal of the second fluorophore to be quenched,
b) contacting the detection solution with the mark under conditions suitable for hybridization of one of the two detection nucleic acid strands with the mark nucleic acid,
c) observing a fluorescence which is emitted by the mark,
and
d) determining the authenticity of the object when (i) the observed fluorescence corresponds to at least one expected first property of the first fluorescence signal and (ii) at least one expected second property of the second fluorescence signal cannot be observed.

2. Method according to claim 1, wherein the first fluorophore and the first quencher are bound in the region of one end of the detection nucleic acid.

3. Method according to any of the preceding claims, wherein the second fluorophore and the second quencher are bound in the region of one end of the reference nucleic acid.

4. Method according to any of the preceding claims, wherein step d) is carried out within 60 seconds after step b).

5. Method according to any of the preceding claims, wherein steps b) and c) are carried out at ambient temperature.

6. Method according to any of the preceding claims, wherein the detection nucleic acid has a hairpin structure in which the detection nucleic acid has two branches that are complementary to each other.

7. Method according to any of the preceding claims, wherein the reference nucleic acid has a hairpin structure in which the reference nucleic acid strands have two branches that are complementary to each other.

8. Method according to any of the preceding claims, wherein step c) comprises illuminating the mark with light of a predefined wavelength range.

9. Method according to any of the preceding claims, wherein the mark is applied to a light-reflecting base.

10. Method according to any of the preceding claims, wherein the detection solution is applied to a single detection field of the mark, wherein said detection field contains the mark nucleic acid.

11. Method according to any of the preceding claims, wherein the detection solution is applied to the mark by use of a pen.

12. Method according to any of the preceding claims, wherein the expected first property is a predefined first minimum intensity within a first wavelength range and the expected second property is a predefined second minimum intensity within a second wavelength range.

13. Method according to any of the preceding claims, wherein the expected first property is a predefined decaying behavior of the first fluorescence signal and the expected second property is a predefined decaying behavior of the second fluorescence signal.

14. Method according to any of the preceding claims, wherein the second fluorophore has a higher intensity than the first fluorophore at a predefined decay time.

15. Method according to any of the preceding claims, wherein the mark contains at least one further nucleic acid in addition to the mark nucleic acid.

16. Method according to any of the preceding claims, wherein a printing ink containing the mark nucleic acid and/or the further nucleic acid is used as the mark.

17. Method according to any of the preceding claims, wherein the mark is applied in a printing process to the object to be marked or to a label which is preferably self-adhesive.

18. Pen containing a detection solution, wherein said detection solution contains an at least in sections double-stranded detection nucleic acid in which one of the two detection nucleic acid strands is at least in sections complementary to a predefined mark nucleic acid, with a first fluorophore being bound to one detection nucleic acid strand and a first quencher being bound to the other detection nucleic acid strand at a distance which causes a first fluorescence signal of the first fluorophore to be quenched, and wherein a hybridization of the detection nucleic acid strand with the mark nucleic acid abolishes the spatial relationship between the quencher and the first fluorophore, and wherein the detection solution further contains an at least in sections double-stranded reference nucleic acid whose reference nucleic acid strands are complementary to neither the mark nucleic acid nor either of the two detection nucleic acid strands, with a second fluorophore which is different from the first fluorophore being bound to one reference nucleic acid strand and a second quencher being bound to the other reference nucleic acid strand at a distance which causes a second fluorescence signal of the second fluorophore to be quenched.

## Revendications

1. Procédé pour authentifier des objets dotés d'une marque, ladite marque comprenant un acide nucléique de marquage, comprenant :
a) l'utilisation d'une solution de détection contenant
a1) un acide nucléique de détection double brin au moins par sections dans lequel l'un des deux brins de l'acide nucléique de détection est au moins par sections complémentaires de l'acide nucléique de marquage, un premier fluorophore étant lié à un brin de l'acide nucléique de détection et un premier extincteur de fluorescence étant lié à l'autre brin de l'acide nucléique de détection à une distance qui provoque l'extinction d'un premier signal de fluorescence du premier fluorophore, et ladite hybridation du brin de l'acide nucléique de détection avec l'acide nucléique de marquage supprimant la relation spatiale entre l'extincteur de fluorescence et le premier fluorophore ;
a2) un acide nucléique de référence double brin au moins par sections dont les brins d'acide nucléique de référence ne sont complémentaires ni de l'acide nucléique de marquage, ni de l'un des deux brins de l'acide nucléique de détection, un second fluorophore, qui est différent du premier fluorophore, étant lié à un brin de l'acide nucléique de référence et un second extincteur de fluorescence étant lié à l'autre brin de l'acide nucléique de référence à une distance qui provoque l'extinction d'un second signal de fluorescence du second fluorophore,
b) la mise en contact de la solution de détection avec la marque dans des conditions appropriées pour l'hybridation d'un des deux brins de l'acide nucléique de détection avec l'acide nucléique de marquage,
c) l'observation d'une fluorescence qui est émise par la marque, et
d) la détermination de l'authenticité de l'objet lorsque (i) la fluorescence observée correspond à au moins une première propriété attendue du premier signal de fluorescence et (ii) au moins une seconde propriété attendue du second signal de fluorescence ne peut pas être observée.

2. Procédé selon la revendication 1, dans lequel le premier fluorophore et le premier extincteur de fluorescence sont liés dans la région d'une extrémité de l'acide nucléique de détection.

3. Procédé selon l'une quelconque des revendications précédentes, dans lequel le second fluorophore et le second extincteur de fluorescence sont liés dans la région d'une extrémité de l'acide nucléique de référence.

4. Procédé selon l'une quelconque des revendications précédentes, dans lequel l'étape d) est réalisée dans les 60 secondes qui suivent l'étape b).

5. Procédé selon l'une quelconque des revendications, dans lequel les étapes b) et c) sont réalisées à température ambiante.

6. Procédé selon l'une quelconque des revendications précédentes, dans lequel l'acide nucléique de détection a une structure en épingle à cheveux dans laquelle l'acide nucléique de détection comprend deux bras qui sont complémentaires l'un de l'autre.

7. Procédé selon l'une quelconque des revendications précédentes, dans lequel l'acide nucléique de référence a une structure en épingle à cheveux dans laquelle l'acide nucléique de référence a deux bras qui sont complémentaires l'un de l'autre.

8. Procédé selon l'une quelconque des revendications précédentes, dans lequel l'étape c) comprend l'éclairage de la marque avec une lumière d'une plage de longueur d'onde prédéfinie.

9. Procédé selon l'une quelconque des revendications précédentes, dans lequel la marque est appliquée sur une base réfléchissant la lumière.

10. Procédé selon l'une quelconque des revendications précédentes, dans lequel la solution de détection est appliquée sur un champ de détection unique de la marque, ledit champ de détection contenant l'acide nucléique de marquage.

11. Procédé selon l'une quelconque des revendications précédentes, dans lequel la solution de détection est appliquée sur la marque en utilisant un stylo.

12. Procédé selon l'une quelconque des revendications précédentes, dans lequel la première propriété attendue est une première intensité minimale prédéfinie comprise dans une première plage de longueur d'onde et la seconde propriété attendue est une seconde intensité minimale prédéfinie comprise dans une seconde plage de longueur d'onde.

13. Procédé selon l'une quelconque des revendications précédentes, dans lequel la première propriété attendue est un comportement de décroissance prédéfini du premier signal de fluorescence et la seconde propriété attendue est un comportement de décroissance prédéfini du second signal de fluorescence.

14. Procédé selon l'une quelconque des revendications précédentes, dans lequel le second fluorophore possède une intensité supérieure à celle du premier fluorophore à un à un temps de décroissance prédéfini.

15. Procédé selon l'une quelconque des revendications précédentes, dans lequel la marque comprend au moins un autre acide nucléique en plus de l'acide nucléique de marquage.

16. Procédé selon l'une quelconque des revendications précédentes, dans lequel une encre d'impression contenant l'acide nucléique de marquage et/ou l'autre acide nucléique est utilisée en tant que marque.

17. Procédé selon l'une quelconque des revendications précédentes, dans lequel la marque est appliquée dans un procédé d'impression sur l'objet à marquer ou sur une étiquette qui est de préférence auto-adhésive.

18. Stylo contenant une solution de détection, dans lequel ladite solution de détection contient un acide nucléique de détection double brin au moins par sections dans lequel l'un des brins de l'acide nucléique de détection est au moins par sections complémentaires d'un acide nucléique de marquage prédéfini, un premier fluorophore étant lié à un brin de l'acide nucléique de détection et un premier extincteur de fluorescence étant lié à l'autre brin de l'acide nucléique de détection à une distance qui provoque l'extinction d'un premier signal de fluorescence du premier fluorophore, et dans lequel une hybridation du brin de l'acide nucléique de détection avec l'acide nucléique de marquage supprime la relation spatiale entre l'extincteur de fluorescence et le premier fluorophore, et dans lequel la solution de détection comprend en outre un acide nucléique de référence double brin au moins par sections dont les brins d'acide nucléique de référence ne sont complémentaires ni de l'acide nucléique de marquage, ni des deux brins de l'acide nucléique de détection, avec un second fluorophore, qui est différent du premier fluorophore, étant lié à un brin de l'acide nucléique de référence et un second extincteur de fluorescence étant lié à l'autre brin de l'acide nucléique de référence à une distance qui provoque l'extinction d'un second signal de fluorescence du second fluorophore.
